(19) Europäisches Patentamt
European Patent Office
Office européen des brevets

(11)   **EP 1 435 390 B1**

(12)   **EUROPEAN PATENT SPECIFICATION**

(45) Date of publication and mention
of the grant of the patent:
**11.11.2009 Bulletin 2009/46**

(51) Int Cl.:
*C12P 19/14* (2006.01)          *C08L 3/02* (2006.01)
*C12R 1/07* (2006.01)

(21) Application number: **03250022.5**

(22) Date of filing: **06.01.2003**

(54) **A process for hydrolyzing starch without ph adjustment**

Verfahren zur Hydrolyse von Stärke ohne pH-Anpassung

Procédé d'hydrolyse de l'amidon sans ajustement du pH

(84) Designated Contracting States:
**AT BE BG CH CY CZ DE DK EE ES FI FR GB GR HU IE IT LI LU MC NL PT SE SI SK TR**

(43) Date of publication of application:
**07.07.2004 Bulletin 2004/28**

(73) Proprietor: **GENENCOR INTERNATIONAL, INC.**
**Palo Alto, California 94304 (US)**

(72) Inventors:
• **Shetty, Jayarama K.**
**Pleasanton,**
**California 94566 (US)**
• **Singley, Eric C.**
**Edwardsburg,**
**Michigan 49112 (US)**
• **Strohm, Bruce A.**
**Goshen,**
**Indiana 46528 (US)**

(74) Representative: **Kiddle, Simon John et al**
**Mewburn Ellis LLP**
**33 Gutter Lane**
**London**
**EC2V 8AS (GB)**

(56) References cited:
**WO-A-92/19744**

• **KANNO M: "A BACILLUS ACIDOCALDARIUS ALPHA-AMYLASE THAT IS HIGHLY STABLE TO HEAT UNDER ACIDIC CONDITIONS" AGRICULTURAL AND BIOLOGICAL CHEMISTRY, JAPAN SOC. FOR BIOSCIENCE, BIOTECHNOLOGY AND AGROCHEM. TOKYO, JP, vol. 50, no. 1, 1986, pages 23-31, XP002028773 ISSN: 0002-1369**
• **PATENT ABSTRACTS OF JAPAN vol. 1998, no. 10, 31 August 1998 (1998-08-31) -& JP 10 136979 A (NAGASE SEIKAGAKU KOGYO KK), 26 May 1998 (1998-05-26)**

**Description**

FIELD OF THE INVENTION

The invention relates to the processing of polysaccharides, such as starch, to produce starch hydrolysates. Specifically, the invention relates to starch hydrolysis processes that do not require a secondary liquefaction step or pH adjustments before and after liquefaction.

BACKGROUND OF THE INVENTION

Grains such as corn have long been used as a source of starch. One of the well-known methods of separating and purifying starch for use in industrial processes is the wet-milling process. This method has developed into a highly specific and integrated system designed to separate the major components of a grain kernel as completely as possible (see Stanley A. Watson, Starch: Chemistry & Technology, Vol. II, Industrial Aspects, Academic Press, New York, 1967, pp. 30-51).

In general, starch conversion processing consists of liquefaction of a granular starch slurry to produce dextrins and saccharification of the liquefied starch into dextrose. Additional processing may include purification and isomerization of dextrose to produce glucose or other sugars, such as fructose.

To liquefy granular starch, heat is applied to the granular starch slurry to disrupt the insoluble starch granules to produce a water-soluble starch solution which is then liquefied by adding an enzyme, such as an $\alpha$-amylase. Typically, the enzymatic liquefaction process involves treatment of the about pH 3.5 to 5.0 granular starch slurry with a base (such as calcium hydroxide, sodium hydroxide or sodium carbonate) to adjust the pH to between about 6.0 and 6.5, the optimum pH range of commonly used $\alpha$-amylases, such as an $\alpha$-amylase derived from *Bacillus licheniformis*. The adjusted suspension may be pumped through a direct steam injection cooker (jet cooker) to raise the temperature to between about 105°-110°C for low temperature liquefaction. In high temperature liquefaction the pH adjustment occurs just after a jet cooker phase at a temperature of about 140-155° C. Following jet cooking, the cooked slurry is cooled to the secondary liquefaction temperature, the pH is adjusted to a range favorable to the selected $\alpha$-amylase, and then the $\alpha$-amylase is added. Alternatively, the starch suspension and added $\alpha$-amylase may be held at a temperature of about 80-100°C to partially hydrolyze the starch granules, and this partially hydrolyzed starch suspension then is pumped through a jet cooker at temperatures in excess of about 105°C to thoroughly gelatinize any remaining granular structure.

Secondary liquefaction at a pH of about 5.5 to 6.0 is carried out to allow the $\alpha$-amylase to continue hydrolysis and reduce the dextrins produced during primary liquefaction. The secondary liquefaction step generally is carried out for about 90 to 120 minutes after cooling the primary liquefact to approximately 95° $\pm$ 5°C. Processing time for liquefaction is selected to produce a DE of approximately 10-12. Dextrose equivalent (DE) is the industry standard for measuring the concentration of total reducing sugars, calculated as glucose on a dry weight basis. Unhydrolyzed granular starch has a DE of virtually zero, whereas the DE of glucose is defined as 100.

Liquefaction temperatures depend upon the source of the $\alpha$-amylase. Alpha-amylases produced by wild-type strains of *B. licheniformis* are preferred by the industry because they are thermostable under typical jet cooking temperatures. The $\alpha$-amylases are thermostabilized with, for example, starch and calcium ions, and generally the starch slurry is adjusted to pH values above about 6 to protect against rapid inactivation at typical liquefaction temperatures. The high pH requirement results in undesirable by-products, e.g., maltulose which ultimately lowers glucose yields.

Kanno M, Agricultural and Biological Chemistry, Japan Soc. for Bioscience, Biotechnology and Agrochem. Tokyo, JP, vol 50, no. 1 1986, pages 23-31 reports a Bacillus acidocaldarius $\alpha$-amylase that is heatstable under acidic conditions.

The pH of the starch slurry suspension from the wet milling stage is about 3.8 to 4.8, and is adjusted upward by the addition of acid neutralizing chemicals, which are removed later by ion-exchange refining of the final starch conversion product. If the liquefact undergoes further processing, such as saccharification utilizing glucoamylase, a pH of 4.0-4.5 is required; therefore, the pH is adjusted again down from about pH 5.5-6.0.

Even though the liquefaction processes employing alpha-amylase currently function to meet the starch processors' operational and performance demands, areas for improvement still remain.

The present invention provides a novel single stage liquefaction process for starch using an acid-stable, thermostable $\alpha$-amylase derived from a selected strain of *Bacillus acidocaldarius* that enables liquefaction without an adjustment of the pH.

SUMMARY OF THE INVENTION

A novel, low temperature starch conversion process comprises a single liquefaction step using a novel acid-stable, thermostable $\alpha$-amylase from a selected strain of *Bacillus acidocaldarius*. The process:

1. Does not require thermostabilizing the enzyme by addition of calcium to the starch slurry;

2. Does not require pH adjustment of the starch slurry prior to enzymatic liquefaction;

3. Operates at a single pH for liquefaction and optional saccharification; and

4. Does not require inactivation of the α-amylase in the liquefact prior to the addition of optional saccharification enzymes when it is desired to convert dextrins to glucose.

Also described herein is, a low temperature starch conversion process with comprises primary and secondary liquefaction steps using low dosages of an acid-stable, thermostable α-amylase from a selected strain of *Bacillus acidocaldarius*. In addition to requiring low enzyme dosages, the process;

1. Does not require thermostabilizing the enzyme by the addition of calcium to the starch slurry;

2. Does not require pH adjustment of the starch slurry prior to enzymatic liquefaction;

3. Operates at a single pH for liquefaction and optional saccharification; and

4. Does require thermo-inactivation of the α-amylase liquefact when conventional temperatures are used during secondary liquefaction prior to the addition of optional saccharification enzymes when it is desired to convert dextrins to glucose.

In another embodiment of the present invention, a high temperature starch conversion process comprises a single liquefaction step using a novel acid-stable, thermostable α-amylase from a selected strain of *Bacillus acidocaldarius*. The process:

1. Does not require thermostabilizing the enzyme by addition of calcium to the starch slurry;

2. Does not require pH adjustment of the starch slurry prior to enzymatic liquefaction;

3. Operates at a single pH for liquefaction and optional saccharification; and

4. Does require thermo-inactivation of the α-amylase in the liquefact prior to the addition of optional saccharification enzymes when it is desired to convert dextrins to glucose.

The novel starch conversion processes utilize fewer chemicals, enable fewer processing steps, operate at a single pH, and can take less time to produce a liquefact with a commercially acceptable DE value and suitable for further optional saccharification. Additionally, the processes produce fewer undesirable by-products during liquefaction, due at least in part to eliminating or reducing typical chemical additions and conducting liquefaction at a reduced pH.

Without wishing to be bound by theory, it is believed that the novel α-amylase is inactivated, consumed, or in some way altered by the end of one of the low temperature liquefaction process thereby enabling saccharification to proceed without the thermo-inactivation utilized in the other embodiments.

Accordingly in one aspect the invention provides a single liquefaction step process for liquefying starch comprising: (a) contacting an aqueous slurry or solution of the starch having a pH between 3.0 and 5.0 with a thermostable, acid-stable alpha-amylase obtained by culturing *Bacillus acidocaldarius* , the contacting occurring at an elevated temperature from 90-155°C without adjusting the pH of the slurry or solution of the starch; and (b) producing a liquefact having a pH of 4.0 to 4.5 and dextrose equivalent (DE) of 10-12.

The invention also provides a single liquefaction step process for liquefying a starch slurry comprising the following acts: (a) providing a thermostable, acid-stable α-amylase prepared from a *Bacillus acidocaldarius* species; (b) adding the alpha-amylase to a starch slurry having a pH between 3.0-5.0; and (c) heating a mixture obtained from step (b) to at least 90°C until a starch liquefact having a pH of 4.0-4.5 and a DE of 10-12 DE is obtained wherein acts (b) and (c) occur without adjusting the pH of the starch slurry.

BRIEF DESCRIPTION OF THE DRAWINGS

FIG. 1A is a prior art low temperature starch liquefaction process.

FIG. 1B is a prior art high temperature starch liquefaction process.

FIGS. 2A, 2B, and 2C are, respectively, a single jet cooking starch liquefaction process embodying principles of the present invention; a high temperature, double jet cooking, starch liquefaction process embodying principles of the present invention; and a low temperature, double jet cooking, starch liquefaction process.

FIG. 3 is a graph showing that conventional alpha-amylase enzymes continue to hydrolyze starch in a secondary liquefaction step, while the KSTM #2037 alpha-amylase of the present invention does not hydrolyze starch in a secondary liquefaction step.

FIG. 4 is a graph showing that only the KSTM #2037 alpha-amylase of the present invention produces a DE of at least 10 after one hour.

Fig. 5 is a graph showing that adding KSTM #2037 alpha-amylase just prior to saccharification decreases glucose yield in saccharification.

Fig. 6 is a graph showing the effect of liquefaction temperatures on KSTM #2037.

Fig. 7 is a chart showing that KSTM #2037 alpha-amylase liquefacts enable production of at least 95% glucose during saccharification.

Fig. 8 is a graph showing a carbohydrate profile of a KSTM #2037 liquefact.

DETAILED DESCRIPTION OF THE INVENTION

The invention comprises the discovery that an acid-stable, thermostable α-amylase from a selected strain of *Bacillus acidocaldarius* enables the conversion of starch to glucose in a single liquefaction step without pH adjustment of the starch slurry, and further enables optional saccharification steps to proceed without a pH adjustment to acceptable saccharification enzyme pH ranges. Additionally, in at least one embodiment of the invention, there is no need to inactivate the *Bacillus acidocaldarius* α-amylase prior to saccharification.

Definitions

"Alpha-amylase" (α-amylase) means an enzyme which cleaves or hydrolyzes the internal α (1-4) glycosidic bonds in starch largely at random to produce α 1-2 bonds resulting in smaller molecular weight maltodextrin, e.g., in starch, high molecular weight amylopectin or amylose polymers are hydrolyzed to oligosaccharides. Suitable α-amylases are the naturally occurring α-amylases as well as recombinant or mutant amylases derived from *Bacillus acidocaldarius*.

"Granular starch" or "starch granules" means a water-insoluble component of edible grains which remains after removal of the hull, fiber, protein, germ, and other soluble substances through the steeping, mechanical cracking, separations, screening, countercurrent rinsing and centrifugation steps typically used in a grain wet-milling process. Granular starch comprises intact starch granules containing almost exclusively packed starch molecules (i.e., amylopectin and amylose). When corn is processed, the granular starch component comprises about 99% starch; the remaining 1% being comprised of protein, ash, fiber and trace components tightly associated with the granules. The packing structure of granular starch retards the ability of α-amylase to hydrolyze starch. Gelatinization of the starch is utilized to disrupt the granules to form a soluble starch solution and facilitate enzymatic hydrolysis.

"Liquefaction" or "liquefy" means a process by which starch is converted to shorter chain and less viscous dextrins. Generally, this process involves gelatinization of starch simultaneously with or followed by the addition of α-amylase. In commercial processes, it is preferred that the granular starch is derived from a source comprising corn, wheat, milo, sorghum, rye potato etc. However, the present invention applies to any grain starch source which is useful in liquefaction, e.g., any other grain or vegetable source known to produce starch suitable for liquefaction. The temperature range of the liquefaction is generally any liquefaction temperature which is known to be effective in liquefying starch. The temperature of the starch is between about 80°C to about 115°C, or from about 100 to about 110°C, or from about 105 to about 108°C for low temperature liquefaction. High temperature liquefaction temperatures are between approximately 140-145°C. According to the single step liquefaction processes of the invention, starch is contacted with the α-amylase at a temperature of 90-155°C (as in claim 1) or the starch/α-amylase mixture is heated to at least 90°C (as in claim 9).

"Saccharification" means the conversion of the liquid product (liquefact), in this case, the hydrolysis of the soluble dextrins to dextrose monomers using enzymes such as glucoamylase. Saccharification products are, for example, glucose and other saccharides such as disaccharides and trisaccharides.

"Starch solution," means the water-soluble gelatinized starch which results from heating granular starch. Upon heating of the granules to above about 72°C, granular starch dissociates to form an aqueous mixture of loose starch molecules. This mixture comprises, for example, about 75% amylopectin and 25% amylose in yellow dent corn, and forms a viscous solution in water. In commercial processes to form glucose or fructose, it is the starch solution, or starch slurry, which is liquefied to form a soluble dextrin solution.

"Steep liquor" means a liquid which is drawn from steeped grain kernels during the steeping process. The steep liquor contains a significant portion of the soluble components of the grain.

The α-amylases suitable for practicing the present invention exhibit the characteristics of the KSTM #2037 α-amylase described in Japanese Patent Application with Publication No. JP10136979, published on May 26, 1998, titled "Novel Acidic Alpha-amylase and Its Production". After the fermentation, the microbial cells were removed by conventional means, leaving the extracellular enzyme in solution. The enzyme containing solution was then concentrated using ultrafiltration and freeze-dried. The activity of the resulting enzyme preparation was found to be 2170 ASAA units/g in Lot Number 1, and 2520 ASAA units/g in Lot Number 2.

The α-amylase activity of the KSTM #2037 α-amylase was measured by determining the hydrolysis of soluble potato starch as described in JP10136979. In a typical run, 10 ml of 1% potato starch solution (pH 4.5 acetate buffer) in an 8 x 180 mm test tube is placed in a constant temperature bath maintained at 40°C for more than 5 minutes. One ml of properly diluted sample is added with agitation and the mixture is incubated for 10 minutes in the constant temperature bath. After 10 minutes, the enzyme reaction is interrupted by rapidly placing 1 ml of the reaction mixture into 10 ml of N/10 HCl. Then, 0.5 ml of the resulting solution is mixed with 10 ml of an iodine solution, and the mixture is shaken well. After two minutes the optical density is determined by a photoelectric calorimeter at 660 millimicrons, using 10 mm cuvettes. The activity unit is calculated using the following equation:

$$AASA = \frac{S-S°}{S \ 10} \ x \ 100 \ x \ n$$

where

S:     optical density obtained without enzyme
S°:    optical density obtained for the sample
n:     dilution of the sample

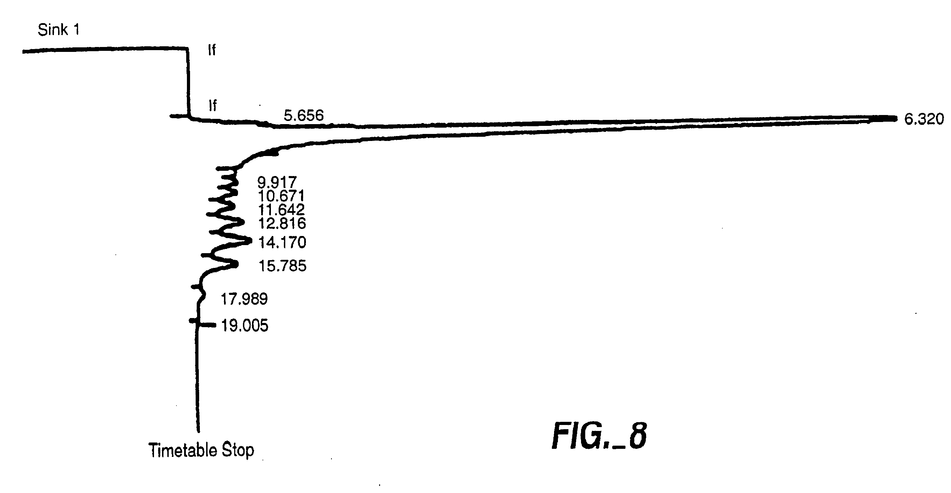

One Acid Stable Alpha Amylase Unit (ASAA Unit) is defined as that activity of enzyme which causes 1% blue value reduction of 1% potato starch solution at 40°C, for one minute. A unit of the diluted sample for purposes of this application generally is between approximately 2 and 5 ASAA. The novel $\alpha$-amylase described above acts to hydrolyze starch substrate to form, mainly, maltopentaose and maltohexaose. Figure 8 illustrates a carbohydrate profile of the novel KSTM #2037 $\alpha$-amylase, 2037, determined by BioRad HPX Column.

PRIOR ART PROCESSES

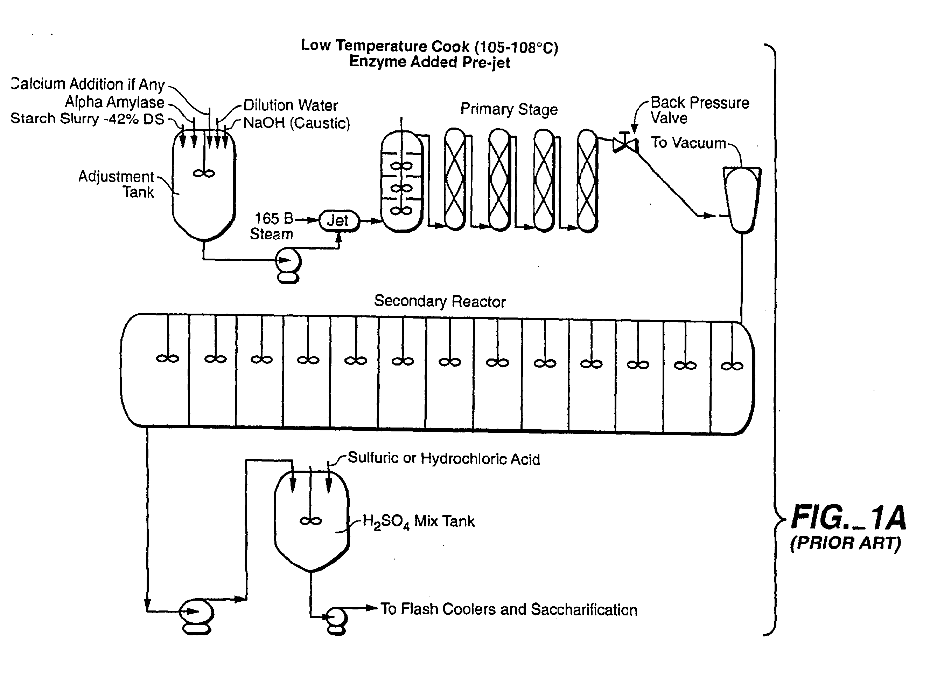

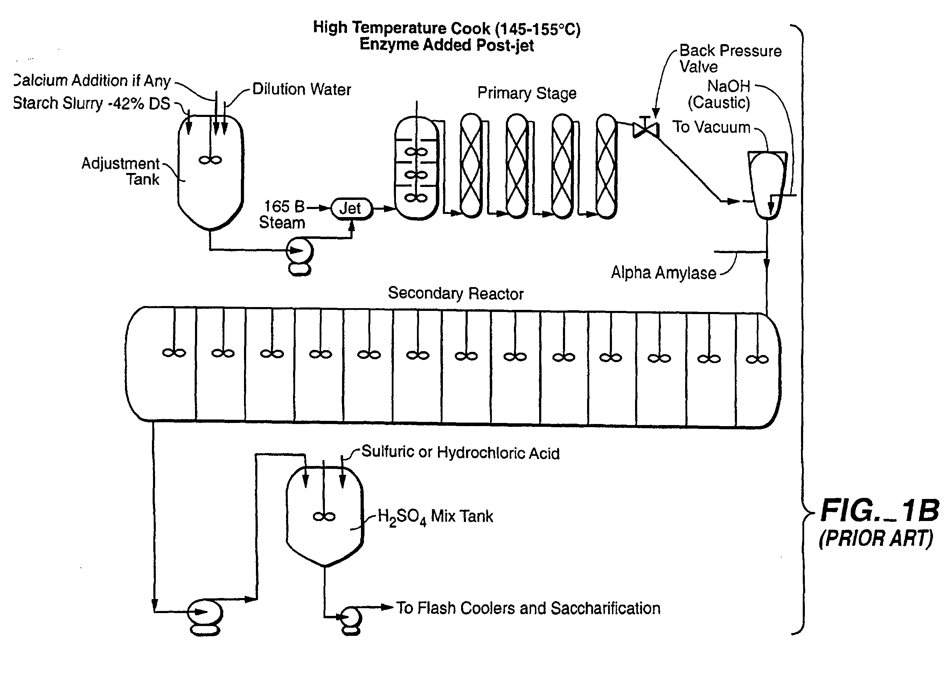

Figure 1A illustrates a prior art low temperature starch conversion process, and Figure 1B illustrates a prior art high temperature starch conversion process. In Figure 1b, a refined starch slurry having a pH between about 3.8 and 4.8 is transferred to a tank and pumped through a steam jet cooker to raise the temperature to about between 140-155°C for a minimum of 20 seconds. The heated mixture then enters the primary liquefaction reactor where it is held at about 140-155°C for approximately 10 minutes to allow hydrolysis and the production of dextrins and starch hydrolysates. The liquefact is then transferred through a flash cooler, and treated to raise the pH to between about 5.5-6.0 by adding calcium hydroxide and/or sodium hydroxide. An $\alpha$-amylase optimally active at about pH 5.5-6.0 and derived from, for example, *Bacillus licheniformis*, is added after the addition of the treating substances and the resulting suspension is sent to a secondary liquefaction reactor where the dextrins are reduced in size. The temperature in the secondary liquefaction reactor is generally about 93-95°C, and the holding time is about 60-120 minutes, or until a target DE is reached.
In low temperature liquefaction, as shown in Fig. 1A, the starch slurry is adjusted from about pH 3.5 to 5.0 to about pH 5.5 to 6.0 using calcium or sodium hydroxide. The slurry then is pumped through a steam jet cooker which utilizes a temperature of approximately 104-108°C in the jet cooker. The heated mixture then is held for about 5 minutes in the pressurized primary liquefaction reactor. Next, the mixture passes through a flash cooler and into the secondary liquefaction reactor where it is maintained at about 90-100°C for approximately 60-120 minutes, or until the target DE is reached.
In either high or low temperature conventional, commercial liquefaction procedures, the commercial $\alpha$-amylases continue hydrolysis during a secondary liquefaction step to reduce the size of the dextrins, although a second addition of $\alpha$-amylase may be added to further hydrolyze the starch. The resulting liquefact from such commercial procedures is expected to include, in the absence of further treatment, by-products at least as a result of the chemical additions. Maltulose is a by-product which is produced at liquefaction pH values greater than 6.0. Maltulose is known to lower glucose yields in subsequent saccharification procedures.
As seen in Figs.1A and 1B, the pH of the resulting liquefaction product is again adjusted by adding an acid such as hydrochloric acid, to lower the pH to between 4 to 4.5 prior to the addition of the enzyme used in optional saccharification. The pH adjustment is used to inactivate the conventional $\alpha$-amylase to prevent the possibility of interference during saccharification and primarily to optimize the activity of the saccharification enzyme. The recognized industry standard for resulting dextrose equivalents (DE) following liquefaction is 10-12.

PROCESSES DESCRIBED HEREIN

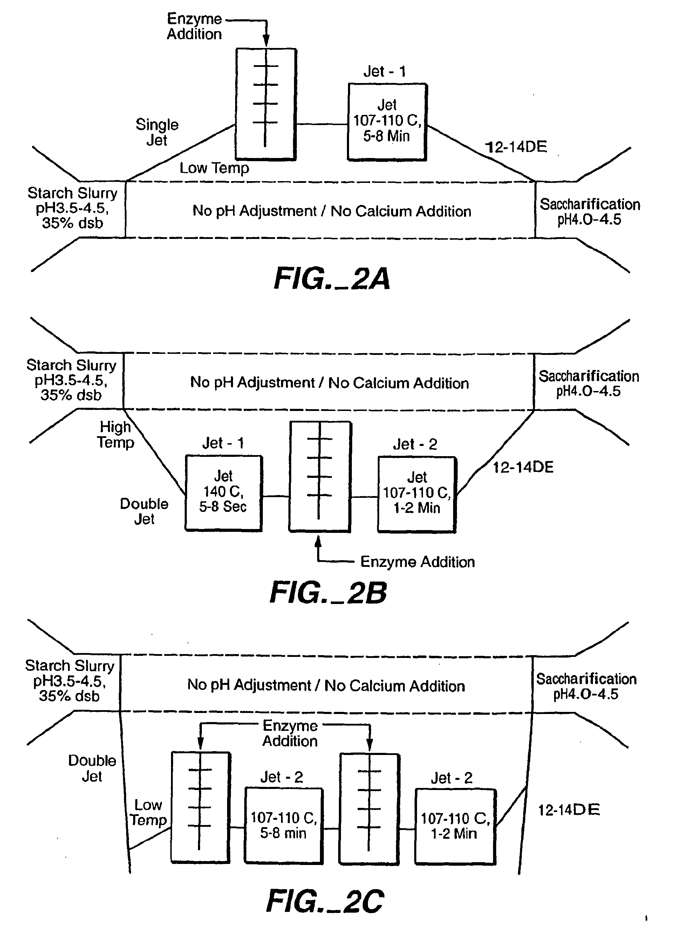

Figures 2A and 2B, illustrate the novel liquefaction processes utilizing an α-amylase from *Bacillus acidocaldarius* constituting the present invention. The processes do not require (1) an initial pH adjustment of the starch slurry prior to addition of the KSTM #2037 α-amylase, (2) the initial addition of calcium salts; (3), the secondary liquefaction step; and (4) the second pH adjustment prior to optional saccharification. All of the processes shown in Figs. 2A, 2B, and 2C are used to liquefy a starch slurry having about 30 - 40% ds and a pH of about 3.5 to 5.0.

In the single jet cooker low temperature liquefaction process shown in Fig. 2A, the KSTM #2037 α-amylase is added to the starch slurry and passed through a jet cooker maintained at about 105-110°C for about 5-8 minutes. This primary liquefaction step occurs without the addition of calcium, although calcium chloride may be added if desired, and typically results in a DE of about 10-12. The liquefaction product has a pH of approximately 4.0-4.5, which is suitable for saccharification enzymes. Additionally, there is no need to make a pH adjustment to inactivate the KTSM α-amylase which is inactive, deactivated, consumed or in some fashion altered so that it does not substantially affect the selected saccharification enzyme.

In the double jet cooker high temperature liquefaction process shown in Fig. 2B, the starch slurry first passes through a high temperature jet cooker for about 5-8 seconds at about 140-145°C. The high temperature slurry next passes through a flash cooler which reduces the temperature to approximately 95-98°C prior to addition of the KSTM #2037 α-amylase. Hydrolysis continues for approximately 60-90 minutes until a DE of 10-12 is reached. The enzymatic reaction then is thermally terminated by passing the liquefact through a second jet cooker maintained at about 107-110° C for several minutes prior to further processing. The double jet cooker liquefaction process achieves the targeted DE of about 10-12, and also does not require the addition of calcium chloride or pH adjustment for the saccharification enzyme prior to optional saccharification, as described above in connection with low temperature liquefaction.

In the double jet cooker low temperature liquefaction process shown in Fig. 2C, a portion of the KSTM #2037 α-amylase is added to the starch slurry prior to passing into a jet cooker for about 5-8 minutes at about 107-110°C. The liquefied starch is then flash cooled and sent to a tank at about 95-98°C where an additional dose of KSTM #2037 α-amylase is added. Hydrolysis continues until a DE of about 10-12 is reached, at which time the enzymatic reaction is terminated by passing the liquefact through a jet cooker maintained at about 107-110°C for 1-2 minutes prior to further processing. The double jet cooker liquefaction process described above enables lower dosages of the novel KSTM #2037 α-amylase as described in Example 6 below as compared to the dosages used in the single stage process shown in Figure 2A.

The following examples are representative, and are not intended to limit the advantages conferred through the use of the invention. However, one of ordinary skill in the art would be able to substitute conditions, reactors, grains, temperature, enzymes and the like according to the above disclosure.

The following examples performed starch liquefaction using a reactor composed of 50 feet of 0.24 inch diameter (0.21 inch i. d.) stainless steel tubing bent into an approximately 10 inch diameter coil that was 5.5 inches high. The coil was equipped with an 11.5 inch, in-line static mixer (Cole - Parmer #G-04669-60 ) mounted 4 feet from the anterior end of the coil. The posterior end of the coil was equipped with a Swagelok in-line adjustable pressure relief valve (#SS-4CA-5 ) set at a cracking pressure of about 20 psi. Starch slurry was fed to the coil at a rate of app. 70 ml/min with a piston metering pump. The coil was heated by immersion in a glycerol-water bath heated to a temperature of approximately 103-120° C for low temperature liquefaction and approximately 140-155°C for high temperature liquefaction. The temperature of the water bath was maintained using a circulating heater/temperature controller (Fisher Scientific model 7305).

EXAMPLE I

COMPARISON OF COMMERCIAL ALPHA-AMYLASES WITH KSTM #2037 ALPHA- AMYLASES

Four commercial, thermostable α-amylases, listed below, were compared with KSTM #2037 α-amylase, as described below.

  1) SPEZYME™ FRED L. [Genencor International] at 100LU/g ds.
  2) Termamyl™ LC. [Novo Nordisk] at 115 LU/g ds.
  3) Termamyl™ SC. [Novo Nordisk] at 75 LU/g ds.
  4) G-995 [Enzyme Bio-Systems] at 75 LU/g ds.

Each α-amylase was tested using the same starch slurry, but under conditions designed to optimize enzymatic performance. Specifically, for all four of the commercial α-amylases, a 35% dsb starch slurry having a pH of 4.0 was treated to contain 20 ppm calcium and 50 ppm $SO_2$ to thermostabilize the enzymes and adjust the pH to 5.6 The KSTM #2037 α-amylase (2170 ASAA units/g) was tested at the slurry pH of 4.0 without any addition of the calcium or $SO_2$ thermosta-

bilizers. All five resulting slurry/enzyme mixtures were subjected to a low temperature jet liquefaction process, such as shown in Fig. 1, at 107°C for 5 min. The dosages for Termamyl™ SC and G-995 α-amylases were selected to be on an equal wt/wt with the SPEZYME™ FRED α-amylase. The target DE after primary liquefaction for all of the enzymes was between 9 and 12. Following primary liquefaction, liquefacts were collected from all five enzyme samples and divided into aliquots for subsequent testing. Specifically, two saccharification samples (enzyme-killed and non-killed) were prepared from each liquefact and the pH was adjusted, particularly for the four commercial α-amylase samples, to 4.0-4.2 with 6N HCL. These samples are discussed below in connection with saccharification studies in Example 8. Another liquefact sample from each enzyme also was allowed to continue hydrolysis in a secondary liquefaction step.

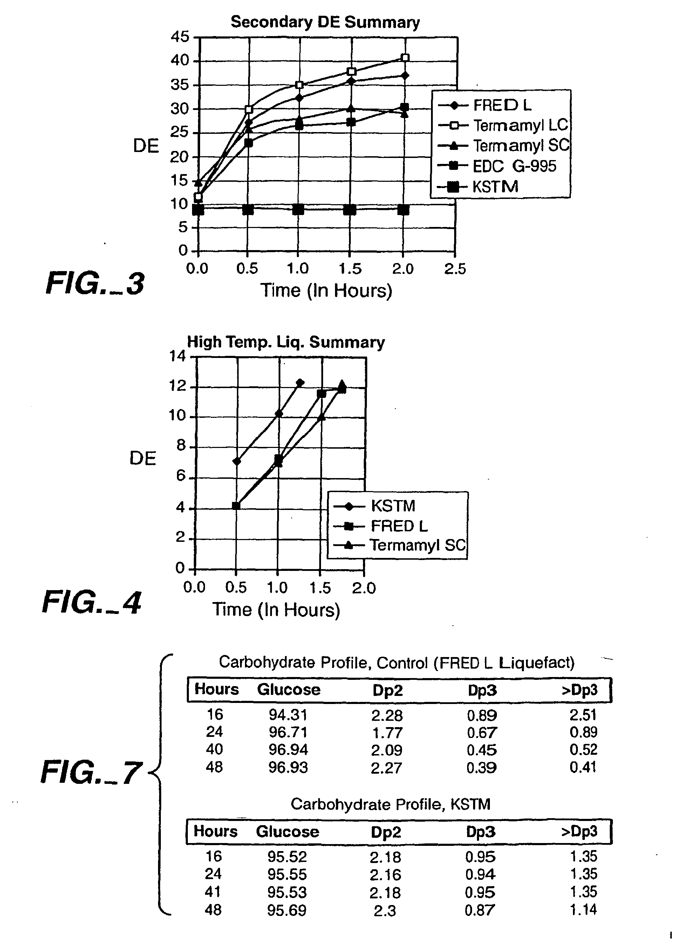

The DE of each of the five hydrolysates continuing in a secondary liquefaction step was then determined at 95°C at selected intervals of time. The DE progression for all of the enzymes is shown in Figure 3. The results shown in Figure 3 demonstrate that, although all of the α-amylases tested produced the target DE amount following primary low temperature, jet liquefaction, only the KSTM #2037 α-amylase did not continue to hydrolyze starch during a secondary liquefaction step. This data suggests that the KSTM #2037 α-amylase liquefaction product does not require treatment to inactivate the enzyme prior to saccharification. On the other hand, Figure 3 demonstrates that all of the conventional α-amylases were still actively hydrolyzing starch up to 2 hours following primary liquefaction, thereby requiring deactivation using chemical additives and pH adjustment prior to optional saccharification procedures.

Two of the three commercial enzymes discussed above, SPEZYME™ FRED L and Termamyl™ SC, also were compared to the KSTM #2037 α-amylase using high temperature liquefaction procedures, such as shown in Figs. 1B or 2B. Specifically, the treated starch slurries prepared as discussed above were sent to a jet cooker and maintained at 140 to 145°C for 5 minutes prior to flashing off to reduce the temperature to 95°C. To test each of the three α-amylases at its optimal performance condition, the addition of the α-amylases to the resulting liquefied slurry was varied. SPEZYME™ FRED α-amylase (10LU/g) and Termamyl™ SC (5 LU/g) alpha-amylase each were added to the liquefied starch slurry pre-treated with 0.5 10N NaOH to adjust the pH to at least 5.6. KSTM #2037 α-amylase (5.0 ASAA units/g ds) was added to an untreated liquefied starch slurry having a pH of 4.0. The three samples were held at 95°C and tested at 30 minute intervals until a target DE of 10 was obtained. Aliquots for subsequent testing were prepared as described above. The results following primary liquefaction are shown in Figure 4. Although all of the tested α-amylases are suitable for the high temperature liquefaction procedure as described, only the KSTM #2037 α-amylase reached the target DE in 1 hour, and this target was reached without adding calcium or conducting a prior pH adjustment. The data shows that use of KSTM #2037 alpha-amylase results in decreased liquefaction times, procedural steps, and chemical and enzyme additions.

The KSTM #2037 α-amylase (2.5 ASAA units/g. ds and 5.0ASAA units/g ds) also was tested at pH 3.5 using the above liquefaction procedure as described in U.S. Pat. No. 3,654,081, The pH 3.5 starch slurry was prepared by suspending 9380 grams of corn starch in 14.2 liters of distilled water containing 50 ppm SO2 to produce a starch concentration of 35% dsb. The results were as follows: 0.5 hours, 8.4-9.0 DE; and 1.0 hours, 11.2-11.6 DE.

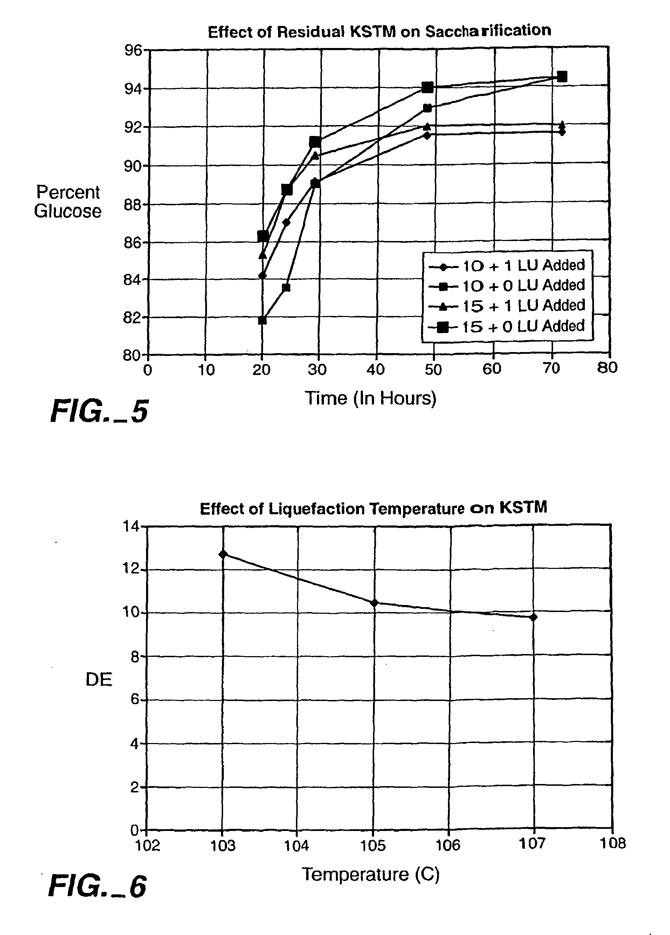

These comparison studies surprisingly suggest that the KSTM #2037 α-amylase enzyme, after primary low temperature liquefaction, for examples as shown in Fig. 2A, is inactivated with respect to its ability to negatively influence saccharification enzymes. This conclusion is supported by saccharification studies using KSTM #2037 α-amylase liquefaction samples with additional KSTM #2037 α-amylase added just prior to saccharification. The results in Figure 5 showed that, as expected, the presence of the KSTM #2037 α-amylase, 10 ASAA units/g, depressed peak dextrose formation in two different saccharification samples.

Without wishing to be bound by any theory, it is believed that KSTM #2037 α-amylase is inactivated, or at the least, altered in some fashion at the end of the low temperature liquefaction process of Fig. 2A so that the enzyme does not substantially interfere with, or influence, saccharification by glucoamylase or other such enzymes.

EXAMPLE 2

CONCENTRATION OF KSTM #2037 α-AMYLASE

The concentration of the KSTM #2037 α-amylase enzyme required to produce at least a 9-10 DE liquefact in the primary jet liquefaction step was studied. In a typical jet-cooking experiment, KSTM #2037 α-amylase was added to 650g of distilled water containing 50 ppm $SO_2$ at concentrations of 100 ASAA and 150 ASAA units/g ds of starch. Starch (350g from Cerestar, USA) was then added and mixed continuously to produce a 35% dsb slurry. The pH of the slurry was then adjusted to pH 4.0. The slurry was passed through a bench cooker maintained at 107°C for 5 minutes then flashed to atmospheric pressure at 95°C for secondary liquefaction. The results are summarized in Table 1, which shows that the 100 ASAA unit/g dose did not produce the targeted DE, and both dosages did not continue to hydrolyze starch under secondary liquefaction conditions.

Table 1

| Effect of Enzyme Concentration on the Final DE of the Liquefaction from the Primary Liquefaction Step | | | | | |
|---|---|---|---|---|---|
| Enzyme dosage Units g. ds. Starch | DE Primary liquefaction step | DE at the Secondary 95°C | | | |
| | 107°C 5 minutes | 30 min | 60 min | 90 min | 120 min |
| 100 ASAA units/g. ds. | 6.2 | 6.49 | 6.11 | 6.37 | 6.47 |
| 150 ASAA units/g. ds. | 9.2 | 9.53 | 9.05 | 9.08 | 9.21 |

EXAMPLE 3

PH EFFECT ON KSTM #2037 $\alpha$-AMYLASE

The effect of the pH of the 35% tsb starch slurry on the liquefaction of starch by KSTM #2037 $\alpha$-amylase was studied under the conditions of Example 1 using 150 ASAA units/g ds. The liquefact recovered after primary liquefaction was held at 95°C with DE determinations made after primary and at 30 minute and 60 minute intervals. The results are shown below in Table 2.

Table 2

| pH of the Starch Slurry | Time at 95°C | DE |
|---|---|---|
| pH 3.5 | 0 After Primary | 9.6 |
| | 30 min. | 9.6 |
| | 60 min. | 9.7 |
| pH 4.0 | 0 After Primary | 9.7 |
| | 30 min. | 9.9 |
| | 60 min. | 10.2 |
| pH 4.5 | 0 After Primary | 9.7 |
| | 30 min. | 9.8 |
| | 60 min. | 9.9 |
| pH 5.0 | 0 After Primary | 9.4 |
| | 30 min. | 9.6 |
| | 60 min. | 9.7 |

The Table 2 results show that the KSTM #2037 $\alpha$-amylase produced a DE of 9-10 under jet cooking conditions at starch slurry pHs between at least about 3.5 to 5.0 thereby showing that the pH of a conventional starch slurry need not be adjusted for liquefaction. It will be clear to those skilled in the art that eliminating this initial pH adjustment also eliminates the need for a second pH adjustment prior to addition of the saccharification enzyme, and particularly in view of the results shown in Figure 3. The results further demonstrate that, using the KSTM #2037 $\alpha$-amylase dosages in Example 1, Fig. 2A, varying pH values between about 3.5 and about 5.0 does not change the results in a secondary liquefaction step, namely the KSTM #2037 $\alpha$-amylase does not continue to hydrolyze starch during a secondary liquefaction step.

EXAMPLE 4

CALCIUM EFFECT ON KSTM #2037 $\alpha$-AMYLASE

KSTM #2037 $\alpha$-amylase was tested to determine the effect of calcium on the KSTM #2037 $\alpha$-amylase during liquefaction at pH 4.0. Calcium generally enhances the stability of thermostable $\alpha$-amylases derived *from Bacillus licheniformis and Bacillus stearothermophilus* used in the industrial starch process. The effect of calcium on the thermostability of KSTM #2037 $\alpha$-amylase under 107° C low temperature jet cooking conditions of starch at pH 4.0 and 150 ASAA units/g is shown in Table 3.

Table 3

| Effect of Added Calcium during Liquefaction of 35% dsb. 50 ppm SO₂ pH 4.0 Starch Slurry Using KSTM #2037 α-Amylase 150 ASAA units/g. ds. | | |
|---|---|---|
| Calcium pmm | Time | DE at 95° |
| 0 | 0, After Primary<br>30 min.<br>60 min. | 9.7<br>9.9<br>10.2 |
| 50 | 0, After Primary<br>30 min.<br>60 min. | 9.8<br>9.6<br>9.9 |
| 100 | 0, After Primary<br>30 min.<br>60 min. | 9.8<br>9.7<br>9.7 |
| 200 | 0, After Primary<br>30 min.<br>60 min. | 9.7<br>9.8<br>9.9 |

Unlike other thermostable conventional $\alpha$-amylases, the results in Table 3 show that calcium does not affect the hydrolytic activity of KSTM #2037 $\alpha$-amylase under jet cooking conditions of the starch at pH 4.0. On the other hand, the results show that, if it is desirable to add calcium for another purpose, the calcium will not interfere with the activity of the KSTM #2037 $\alpha$-amylase. Starches, such as ground corn, contain phytic acid which binds calcium thereby decreasing the performance of conventional $\alpha$-amylases which require calcium addition for stability.

EXAMPLE 5

High Temperature KTSM Alpha-Amylase Process

The high temperature jet-cooking process described in U.S. Pat. No. 3,654,081, was used in this example to liquefy starch using thermostable alpha-amylase. A pH 3.5 starch slurry was prepared by suspending 9380 grams of corn starch in 14.2 liters of distilled water containing 50 ppm $SO_2$ to produce a starch concentration of 35% DSB. The starch slurry was then gelatinized in a pilot plant steam jet-cooker at 140°C with a 5 minute holding loop. The gelatinized starch was then flashed directly into a temperature-regulated vessel maintained at between 95°C-98°C, and KSTM #2037 alpha-amylase was added at a concentration of 2.5 ASAA units/g. dsb. Samples were taken at 30-minute intervals to determine DE production. The enzyme reaction was terminated by heating the liquefied starch at 110°C for 2 minutes. The results are shown below in Table 4.

Table 4

| Dextrose Equivalent of the Liquefied Starch | |
|---|---|
| Liquefaction at 95°C pH | DE Progression |
| | 2.5 ASAA units/g.dsb |
| 30 min.<br>60 min. | 8.99<br>11.22 |
| 110°C for 5 min. Enzyme inactivation step | 13.57 |

Table 4 demonstrates that thermo-inactivation of the KSTM #2037 $\alpha$-amylase should be performed prior to saccharification for processes which utilize liquefaction temperatures of less than and around 95° C. The increase in DE shown in the 110° C inactivation step represents the type of increase that was seen during the low temperature liquefaction process described above, Fig. 2A, and the enzyme is inactivated at the end of the 5 minute loop thereby halting any further increase in the DE level.

EXAMPLE 6

EFFECT OF LIQUEFACTION TEMPERATURE ON KSTM #2037 ALPHA-AMYLASE

KSTM #2037 α-amylase was tested to determine the effects of high liquefaction temperatures. The results shown in Figure 6 demonstrate that the KSTM #2037 α-amylase produced acceptable DE values of more than 9 to about 13 at temperatures between about 103° C and about at least 107° C.

EXAMPLE 7 (Reference Example)

DUAL ENZYME ADDITION

The low temperature jet-cooking process shown in Fig. 2C was used to study the effect of adding a split dosage of the enzyme, i.e. pre-and post-jet cooking of the starch. In Trial A, KSTM #2037 α-amylase at 15 ASAA units/g. ds was added to a 35% tsb starch slurry at pH 3.8-4.0. The mixture was passed through a jet cooker maintained at 107°C for 5 min. At a secondary liquefaction stage (95°C, pH 4.0) an additional amount of the KSTM #2037 α-amylase was added, namely 5 ASAA units/g. dsb. Liquefaction was then continued at 95°C and samples were taken at 30-min. intervals for DE analysis. The above experiment was repeated using KSTM #2037 α-amylase at 30 ASAA units/g. dsb (Trial #B) pre-jet followed by an additional post-jet amount of 5 ASAA units/g. dsb., starch at 95°C. The results are shown below in Table 5.

Table 5

| Effect of Split-Dose with KSTM #2037 α-Amylase on the Liquefaction of Starch at pH 4.0 | | | | | | | |
|---|---|---|---|---|---|---|---|
| Trial | Enzyme dosage, ASAA units/g. dsb. | | DE Progression at 95°C pH 4.0 | | | | |
| | Pre-Jet | Post Jet | 0 | 30 | 60 | 90 | 120 |
| Trial #A | 15 | 5 | 1.81 | 7.02 | 10.08 | 12.35 | 14.48 |
| Trial #B | 30 | 5 | 2.30 | 8.42 | 11.40 | 13.33 | 16.08 |

Comparison of the results in Table 1 and Table 5 shows that, if low enzyme dosages are desired in a low temperature liquefaction process, acceptable DE values may be obtained within one hour of an added secondary liquefaction step (see Figure 2C) with lower dosages of KTSM α-amylase.

The data present in Examples 1 through 7 demonstrate the liquefaction of a starch slurry at a pH between 3.5-5.0 with no added calcium using varying concentrations of KSTM #2037 α-amylase and conventional high and low starch processing temperatures. The data further demonstrate that the KSTM #2037 α-amylase produces acceptable DE amounts in a single low temperature primary liquefaction step at concentrations of about at least 140 ASAA units/g dsb, and in a high temperature single liquefaction step, at concentrations of about 1-10 ASAA units/g. The data also shows that the KSTM #2037 α-amylase may be added in one or more dosages in low temperature liquefaction, and that a first lower dosage of approximately 10 to 35 ASAA units/g followed by a second addition of about 1-10 ASAA units/g may be used to produce acceptable DE amounts in a secondary liquefaction step. In the low temperature liquefaction process described above and shown in Figure 2A, after liquefaction, the enzyme is inactivated, thereby enabling processes such as saccharification to proceed without a further pH adjustment to inactive the enzyme. In the processes of Figures 2B and 2C, the liquefact is treated thermally to inactivate α-amylase if saccharification processes are to be carried out, and the pH need not be adjusted for the saccharification enzyme.

The following examples discuss optional saccharification processes and compare the KSTM #2037 α-amylase liquefacts to conventional α-amylase liquefacts.

EXAMPLE 8

Saccharification

The KSTM #2037 α-amylase hydrolyzed, liquefied starch sample collected and described in the low temperature liquefaction process described in Example 1 was saccharified using glucoamylase (Optidex™ L-400) and glucoamylase containing different concentrations of pullulanase (Optimax™ ). Referring now to Table 7, KSTM #2037 liquefacts were saccharified with the following enzymes: "A" Set using 0.22 units/g of glucoamylase; "B" Set using a mixture of 0.22 units/g of glucoamylase and 0.069 acid stable pullulanase unit/g; "C" Set using a mixture of 0.22 units/g of glucoamylase and 0.147 acid stable pullulanase units/g; "D" Set using a mixture of 0.22 units/g of glucoamylase and 0.330 acid stable pullulanase units/g; and "E" Set using a mixture of 0.22 units of glucoamylase and 0.880 acid stable pullulanase units/g. Saccharification was carried out at 32% ds at 60° without any pH adjustment of the pH 4.0 liquefact. Samples were taken at different intervals of time and the composition of the reaction products was determined by using a standard high pressure liquid chromatographic method under the following conditions:

| | |
|---|---|
| Column | HPX-87 C |
| Mobile phase | Double Distilled Water |
| Temp °C | 80 |
| Detection | RI |
| Concentration | 20 microliter of 3% solution |

The results are shown below in Table 7 which demonstrates that using KTSM alpha-amylase liquefacts for saccharification results in typical dextrose levels. Additional saccharification studies were conducted at 103° C, 105° C, and 107°C using Sets "B" and "D". These additional studies demonstrated attainment of about 95% glucose in 24-48 hours.

Table 7

| Saccharification of KSTM #2037 Liquefied Starch Substrate by Saccharification Enzymes, | | | | | |
|---|---|---|---|---|---|
| pH 4.0, 60°C, 32% dsb | | | | | |
| Sample | Hours | Glucose | Di-sacch | Tri-sacch | Highers |
| "A" Set OPTIDEX L 400 0.22 GAU/g DS 0 ASPU/g DS | 16 | 89.34 | 1.91 | 1.04 | 7.72 |
| | 24 | 92.47 | 2.18 | 1.00 | 4.35 |
| | 39 | 94.22 | 2.74 | 0.98 | 2.06 |
| | 48 | 94.48 | 3.07 | 0.97 | 1.48 |
| "B" Set OPTIMAX 7525 HP 0.22 GAU/g DS 0.069 ASPU/g DS | 16 | 90.00 | 1.96 | 1.13 | 6.92 |
| | 24 | 93.03 | 2.23 | 1.10 | 3.64 |
| | 39 | 94.50 | 2.80 | 1.05 | 1.64 |
| | 48 | 94.62 | 3.16 | 1.02 | 1.20 |
| "C" Set OPTIMAX 6040 0.22 GAU/g DS 0.147 ASPU/g DS | 16 | 90.00 | 1.98 | 1.20 | 6.83 |
| | 24 | 93.19 | 2.19 | 1.17 | 3.45 |
| | 39 | 94.63 | 2.74 | 1.08 | 1.55 |
| | 48 | 94.70 | 3.07 | 1.05 | 1.18 |
| "D" Set OPTIMAX 4060 0.22 GAU/g DS 0.330 ASPU/g DS | 16 | 91.10 | 2.04 | 1.33 | 5.54 |
| | 24 | 93.93 | 2.22 | 1.23 | 2.62 |
| | 39 | 94.88 | 2.74 | 1.08 | 1.30 |
| | 48 | 94.83 | 3.09 | 1.03 | 1.06 |
| "E" Set HIGH DEX 2080 0.22 GAU/g DS 0.880 ASPU/g DS | 16 | 92.63 | 2.09 | 1.45 | 3.83 |
| | 24 | 94.70 | 2.21 | 1.27 | 1.82 |
| | 39 | 95.02 | 2.76 | 1.09 | 1.02 |
| | 48 | 94.92 | 3.06 | 1.04 | 0.85 |

The liquefact from KSTM #2037 $\alpha$-amylase produced glucose syrup having greater than 95% glucose which is an acceptable target level for commercial saccharification processes. A control study was conducted using the SPEZYME™ FRED L enzyme described in Example 1. The liquefact for the control had a DE of 9.07 and was inactivated by lowering the pH to 4.0 to 4.2 at 95° C. The KSTM #2037 and SPEZYME™ FRED L liquefacts were saccharified with OPTIMAX™ 2080 under the same conditions shown in Table 7. The results of the comparison are in Figure 7, which shows that both samples produced greater than 95% glucose yields.

Evaluation of the novel KSTM #2037 $\alpha$-amylase on the liquefaction of starch in the pH range of 3.5 to 5.0 with no added calcium provided at least three different options for operating conditions.

I) Single Jet Low Temperature Process:

As shown in Figure 2a, for example, the novel process involves the addition of KSTM #2037 $\alpha$-amylase (generally approximately at least 140 ASAA units/g. dsb) to a starch slurry, 35% ds at a pH ranging from about as low as 3.0 to about at least 5.0 and passing the mixture through a jet cooker maintained at about 105-110°C for 5-8 min. No calcium need be added to the starch slurry. This single, rapid primary liquefaction step utilizing KSTM #2037 $\alpha$-amylase in the stated concentration range generally results in a targeted DE of 10-12 thereby eliminating the need for a secondary liquefaction step; i.e. 95°C $\pm$ for 90-120 min. The liquefact produced in this process is suitable for saccharification without enzyme inactivation and without a pH adjustment.

II) Double Jet-High Temperature Process

In this process shown in Figure 2b, for example, a starch slurry (35/tsb having a pH as low as 3.0 to about at least 5.0) is first subjected to a high temperature jet cooking process (between about 140-155°C for about 5-8 seconds) and the gelatinized starch is then flashed to atmospheric pressure and maintained at about 95°C-98°C at the above pH. Then, KSTM #2037 $\alpha$-amylase is added in an amount between about 1-10 ASAA units/g ds. and the hydrolysis is continued for about 60-90 min. until a desired DE of 10-12 is reached. The enzyme reaction is then terminated by passing the liquefact through a jet cooker maintained at about 107-110°C for 1-2 min prior to further saccharification processing. The double jet high temperature process utilizes less enzyme than the Single Jet Low Temperature Process to produce a 10-12 DE liquefact, produces such a liquefact in about an hour, utilizes a thermal inactivation step if saccharification is to occur, and does not require a pH adjustment prior to saccharification.

III) Double Jet Low Temperature Process (Comparative Process)

In this process shown in Figure 2c, a portion of the enzyme (about 10-35 ASAA units/g. dsb.) is added to the starch slurry, (pH as low as 3.0 to about at least 5.0) prior to low temperature jet cooking (about 105-110°C for 5-8 min). After the primary liquefaction step, the liquefied starch is flashed to a tank maintained at about 95°C-98°C. An additional dose of KSTM #2037 $\alpha$-amylase is then added (about 1-10 ASAA units/g.dsb) and hydrolysis is continued until a desired DE of 10-12 is reached. The enzyme reaction is then terminated by passing the liquefact through a jet cooker maintained at about 107-110°C for 1-2 minutes prior to further processing. The thermally inactivated liquefact from this process is suitable for saccharification without further treatment.

**Claims**

1. A single liquefaction step process for liquefying starch comprising: (a) contacting an aqueous slurry or solution of the starch having a pH between 3.0 and 5.0 with a thermostable, acid-stable alpha-amylase obtained by culturing *Bacillus acidocaldarius*, the contacting occurring at an elevated temperature from 90-155° C without adjusting the pH of the slurry or solution of the starch; and (b) producing a liquefact having a pH of 4.0 to 4.5 and dextrose equivalent (DE) of 10-12.

2. The process of claim 1 wherein the contacting occurs without adding a calcium salt to the slurry or solution.

3. The process of claim 1 wherein the contacting act further comprises maintaining the contact at the elevated temperature for 5-8 minutes.

4. The process of claim 3 wherein the elevated temperature is 105-110°C.

5. The process of claim 4 further comprising providing at least 150 acid-stable alpha amylase (ASAA) units/g of the thermostable, acid-stable alpha-amylase.

6. The process of claim 1 further comprising, prior to the act of contacting, cooking the aqueous slurry or solution of the starch at between 140-155° C for 5-8 seconds and then reducing the temperature of the cooked slurry or solution to 90-98°C.

7. The process of claim 6 wherein the contacting occurs between the thermostable, acid-stable alpha-amylase and the cooked slurry or solution at 90-98°C for 60-90 minutes.

8. The process of claim 7 further comprising providing 1.0 to 10 ASAA units/g of the thermostable, acid-stable alpha-amylase.

9. A single liquefaction step process for liquefying a starch slurry comprising the following acts:

(a) providing a thermostable, acid-stable $\alpha$-amylase prepared from a *Bacillus acidocaldarius* species;
(b) adding the alpha-amylase to a starch slurry having a pH between 3.0 - 5.0; and
(c) heating a mixture obtained from step (b) to at least 90° C until a starch liquefact having a pH of 4.0-4.5 and a DE of 10-12 DE is obtained wherein acts (b) and (c) occur without adjusting the pH of the starch slurry.

10. The process of claim 9 wherein acts (b) and (c) occur without adding a calcium salt to the starch slurry.

11. The process of claim 9 wherein act (c) occurs at 90-155° C.

12. The process of claim 9 wherein act (c) further comprises heating the mixture for 5-8 minutes.

13. The process of claim 12 wherein the mixture is heated to 105-110°C in act (c).

14. The process of claim 13 wherein act (b) further comprises adding at least 140 ASAA units/g of the thermostable, acid-stable alpha-amylase to the starch slurry.

15. The process of claim 9 further comprising prior to act (b), the act of cooking the starch slurry at between 140-155° C for 5-8 seconds and then reducing the temperature of the cooked slurry or solution to 90-98°C prior to adding the alpha-amylase.

16. The process of claim 15 wherein act (c) further comprises holding the mixture at 90-98° C for 60-90 minutes.

17. The process of claim 16 wherein act (b) further comprises adding 1.0 to 5.0 ASAA units/g of the thermostable, acid-stable alpha-amylase.

18. A starch liquefaction process according to claim 9 wherein step (b) comprises adding about 150 ASAA units/g of the alpha-amylase; and step (c) comprises jet cooking the mixture resulting from step (b) for 5-8 minutes at 105-110° C.

19. A starch liquefaction process according to claim 9 which comprises, prior to step (b) jet cooking the starch slurry at between 140-155° C for 5-8 seconds without adjusting the pH of the starch slurry and lowering the temperature of the cooked slurry to 95-98 C°; and wherein step (b) comprises adding 1.0 to 5.0 ASAA units/g digestible starch (ds) of the alpha-amylase; and wherein step (c) comprises allowing the mixture to undergo hydrolysis for 60-90 minutes to produce a liquefact having a DE of 10-12.

**Patentansprüche**

1. Einstufiges Verflüssigungsverfahren zum Verflüssigen von Stärke, umfassend: (a) das Kontaktieren einer wässrigen Aufschlämmung oder Lösung der Stärke, die einen pH-Wert zwischen 3,0 und 5,0 aufweist, mit einer durch Züchten von Bacillus acidocaldarius erhaltenen hitzebeständigen, säurebeständigen Alpha-Amylase, wobei das Kontaktieren bei einer erhöhten Temperatur von 90 bis 155 °C ohne Einstellen des pH-Werts der Aufschlämmung oder Lösung der Stärke stattfindet; und (b) das Produzieren eines Verflüssigungsprodukts, das einen pH-Wert von 4,0 bis 4,5 und ein Dextrose-Äquivalent (DE) von 10 bis 12 aufweist.

2. Verfahren nach Anspruch 1, worin das Kontaktieren ohne Hinzufügen eines Calciumsalzes zu der Aufschlämmung oder Lösung stattfindet.

3. Verfahren nach Anspruch 1, worin der Vorgang des Kontaktierens weiters das 5 bis 8 Minuten lange Aufrechterhalten des Kontakts bei erhöhter Temperatur umfasst.

4. Verfahren nach Anspruch 3, worin die erhöhte Temperatur 105 bis 110 °C beträgt.

5. Verfahren nach Anspruch 4, weiters umfassend das Bereitstellen von mindestens 150 Einheiten von säurebeständiger Alpha-Amylase (ASAA)/g der hitzebeständigen, säurebeständigen Alpha-Amylase.

6. Verfahren nach Anspruch 1, weiters umfassend das vor dem Vorgang des Kontaktierens stattfindende 5 bis 8 Sekunden lange Kochen der wässrigen Aufschlämmung oder Lösung der Stärke bei einer Temperatur zwischen 140 bis 155 °C und das anschließende Senken der Temperatur der gekochten Aufschlämmung oder Lösung auf 90 bis 98 °C.

7. Verfahren nach Anspruch 6, worin das Kontaktieren zwischen der hitzebeständigen, säurebeständigen Alpha-Amylase und der gekochten Aufschlämmung oder Lösung bei 90 bis 98 °C 60 bis 90 Minuten lang stat tfindet.

8. Verfahren nach Anspruch 7, weiters umfassend das Bereitstellen von 1,0 bis 10 ASAA-Einheiten/g der hitzebeständigen, säurebeständigen Alpha-Amylase.

9. Einstufiges Verflüssigungsverfahren zur Verflüssigung einer Stärkeaufschlämmung, umfassend die folgenden Vorgänge:

   (a) Bereitstellen einer hitzebeständigen, säurebeständigen α-Amylase, die aus einer Bacillus-acidocaldarius-Spezies hergestellt wurde;
   (b) Hinzufügen der Alpha-Amylase zu einer Stärkeaufschlämmung, die einen pH-Wert zwischen 3,0 und 5,0 aufweist; und
   (c) Erhitzen eines aus Schritt (b) erhaltenen Gemisches auf mindestens 90 °C, bis ein Stärkeverflüssigungsprodukt, das einen pH-Wert von 4,0 bis 4,5 und ein DE von 10 bis 12 DE aufweist, erhalten wird, worin die Vorgänge (b) und (c) ohne Einstellen des pH-Werts der Stärkeaufschlämmung stattfinden.

10. Verfahren nach Anspruch 9, worin die Vorgänge (b) und (c) ohne Hinzufügen eines Calciumsalzes zu der Stärkeaufschlämmung stattfinden.

11. Verfahren nach Anspruch 9, worin Vorgang (c) bei 90 bis 155 °C stattfindet.

12. Verfahren nach Anspruch 9, worin Vorgang (c) weiters das 5 bis 8 Minuten lange Erhitzen des Gemisches umfasst.

13. Verfahren nach Anspruch 12, worin das Gemisch in Vorgang (c) auf 105 bis 110 °C erhitzt wird.

14. Verfahren nach Anspruch 13, worin Vorgang (b) weiters das Hinzufügen von mindestens 140 ASAA-Einheiten/g der hitzebeständigen, säurebeständigen Alpha-Amylase zu der Stärkeaufschlämmung umfasst.

15. Verfahren nach Anspruch 9, weiters umfassend den vor Vorgang (b) stattfindenden Vorgang des 5 bis 8 Sekunden langen Kochens der Stärkeaufschlämmung bei einer Temperatur zwischen 140 und 155°C und da s anschließende Senken der Temperatur der gekochten Aufschlämmung oder Lösung auf 90 bis 98 °C vor dem Hinzufügen der Alpha-Amylase.

16. Verfahren nach Anspruch 15, worin Vorgang (c) weiters das 60 bis 90 Minuten lange Halten des Gemisches bei 90 bis 98 °C umfasst.

17. Verfahren nach Anspruch 16, worin Vorgang (b) weiters das Hinzufügen von 1,0 bis 5,0 ASAA-Einheiten/g der hitzebeständigen, säurebeständigen Alpha-Amylase umfasst.

18. Stärkeverflüssigungsvorgang nach Anspruch 9, worin Schritt (b) das Hinzufügen von etwa 150 ASAA-Einheiten/g der Alpha-Amylase umfasst; und Schritt (c) das 5 bis 8 Minuten lange Konvektionskochen des aus Schritt (b) resultierenden Gemisches bei 105 bis 110 °C umfasst.

19. Stärkeverflüssigungsvorgang nach Anspruch 9, der das vor Schritt (b) stattfindende 5 bis 8 Sekunden lange Konvektionskochen der Stärkeaufschlämmung bei einer Temperatur zwischen 140 und 155 °C ohne Einst ellen des pH-Werts der Stärkeaufschlämmung und das Senken der Temperatur der gekochten Aufschlämmung auf 95 bis 98 °C umfasst; und worin Schritt (b) das Hinzufügen von 1,0 bis 5,0 ASAA-Einheiten/g verdaulicher Stärke (ds) der Alpha-Amylase umfasst; und worin Schritt (c) das Ermöglichen einer 60- bis 90-minütigen Hydrolyse des Gemisches umfasst, um ein Verflüssigungsprodukt mit einem DE von 10 bis 12 zu produzieren.

**Revendications**

1. Procédé à étape de liquefaction unique pour liquéfier l'amidon comprenant: (a) mettre en contact une boue ou solution aqueuse de l'amidon d'un pH entre 3,0 et 5,0 avec une alpha-amylase thermostable, stable à l'acide, obtenue en cultivant le _bacillus acidocaldarius,_ la mise en contact ayant lieu à une température élevée de 90-155°C sans ajuster le pH de la boue ou de la solution de l'amidon; et (b) produire un liquéfact d'un pH de 4,0 à 4,5 et un équivalent de dextrose (DE) de 10-12.

2. Procédé selon la revendication 1, dans lequel la mise en contact a lieu sans ajouter de sel de calcium à la boue ou solution.

3. Procédé selon la revendication 1, dans lequel l'acte de mise en contact comprend en outre le maintien du contact à la température élevée pendant 5-8 minutes.

4. Procédé selon la revendication 3, dans lequel la température élevée est de 105-110°C.

5. Procédé selon la revendication 4, comprenant en outre la réalisation d'au moins 150 unités/g d'alpha-amylase stable à l'acide (ASAA) de l'alpha-amylase thermostable, stable à l'acide.

6. Procédé selon la revendication 1, comprenant en outre, avant l'acte de la mise en contact, la cuisson de la boue ou solution aqueuse de l'amidon entre 140-155°C pendant 5-8 secondes suivie de la réduction de la température de la boue ou solution cuite à 90-98°C.

7. Procédé selon la revendication 6, dans lequel la mise en contact a lieu entre l'apha-amylase thermostable, stable à l'acide et la boue ou solution cuite à 90-98°C pendant 60-90 minutes.

8. Procédé selon la revendication 7, comprenant en outre la réalisation de 1,0 à 10 unités/g de ASAA de l'alpha-amylase thermostable, stable à l'acide.

9. Procédé à étape de liquéfaction unique pour liquéfier une boue d'amidon comprenant les actes suivants:

   (a) réaliser une alpha-amylase thermostable, stable à l'acide, préparée à partir d'une espace de _Bacillus acidocaldarius;_
   (b) ajouter l'alpha-amylase à une boue d'amidon ayant un pH entre 3,0-5,0; et
   (c) chauffer un mélange obtenu à l'étape (b) à au moins 90°C jusqu'à ce qu'un liquéfact d'amidon d'un pH de 4,0-4,5 et d'un DE de 10-12 DE soit obtenu, où les actes (b) et (c) ont lieu sans ajuster le pH de la boue d'amidon.

10. Procédé selon la revendication 9, dans lequel les actes (b) et (c) ont lieu sans ajouter de sel de calcium à la boue d'amidon.

11. Procédé selon la revendication 9, dans lequel l'acte (c) a lieu à 90-150°C.

12. Procédé selon la revendication 9, dans lequel l'acte (c) comprend en outre l'échauffement du mélange pendant 5-8 minutes.

13. Procédé selon la revendication 12, dans lequel le mélange est chauffé à 105-100°C dans l'acte (c).

14. Procédé selon la revendication 13, dans lequel l'acte (b) comprend en outre l'ajout d'au moins 140 unités/g de ASAA de l'alpha-amylase thermostable, stable à l'acide, à la boue d'amidon.

15. Procédé selon la revendication 9, comprenant en outre avant l'acte (b), l'acte de cuisson de la boue d'amidon entre 140-155°C pendant 5-8 secondes suivi de la réduction de la température de la boue ou solution cuite à 90-98°C avant l'ajout de l'alpha-amylase.

**16.** Procédé selon la revendication 15, dans lequel l'acte (c) comprend en outre le maintien du mélange à 90-98°C pendant 60-90 minutes.

**17.** Procédé selon la revendication 16, dans lequel l'acte (b) comprend en outre l'ajout de 1,0 à 5,0 unites/g ASAA de l'alpha-amylase thermostable, stable à l'acide.

**18.** Procédé de liquéfaction d'amidon selon la revendication 9, dans lequel l'étape (b) comprend l'ajout d'environ 150 unités/g de ASAA de l'alpha-amylase et l'étape (c) comprend la cuisson à jet du mélange résultant de l'étape (b) pendant 5-8 minutes à 105-110°C.

**19.** Procédé de liquéfaction d'amidon selon la revendication 9, qui comprend, avant l'étape (b), la cuisson à jet de la boue d'amidon entre 140-150°C pendant 5-8 secondes sans ajuster le pH de la boue d'amidon et la réduction de la température de la boue cuite à 95-98°C; et où l'étape (b) comprend l'ajout de 1,0 à 5,0 unités/g de ASAA d'amidon digestible (ds) de l'alpha-amylase; et où l'étape (c) comprend l'amenée du mélange à subir une hydrolyse pendant 60-90 minutes pour produire un liquéfact ayant un DE de 10-12.

**Low Temperature Cook (105-108°C) Enzyme Added Pre-jet**

Calcium Addition if Any
Alpha Amylase
Starch Slurry -42% DS
Dilution Water
NaOH (Caustic)

Adjustment Tank

Primary Stage

Back Pressure Valve

To Vacuum

165 B Steam
Jet

Secondary Reactor

Sulfuric or Hydrochloric Acid

H₂SO₄ Mix Tank

To Flash Coolers and Saccharification

*FIG._1A*
*(PRIOR ART)*

EP 1 435 390 B1

**High Temperature Cook (145-155°C)**
**Enzyme Added Post-jet**

*FIG._1B*
*(PRIOR ART)*

EP 1 435 390 B1

Enzyme
Addition

Jet - 1

Jet
107-110 C,
5-8 Min

Single
Jet

12-14DE

Low Temp

Starch Slurry
pH3.5-4.5,
35% dsb

No pH Adjustment / No Calcium Addition

Saccharification
pH4.O-4.5

**FIG._2A**

Starch Slurry
pH3.5-4.5,
35% dsb

No pH Adjustment / No Calcium Addition

Saccharification
pH4.O-4.5

High
Temp

Jet - 1

Jet
140 C,
5-8 Sec

Jet - 2

Jet
107-110 C,
1-2 Min

12-14DE

Double
Jet

Enzyme Addition

**FIG._2B**

Starch Slurry
pH3.5-4.5,
35% dsb

No pH Adjustment / No Calcium Addition

Saccharification
pH4.O-4.5

Double
Jet

Enzyme
Addition

Jet - 2

107-110 C,
5-8 min

Jet - 2

107-110 C,
1-2 Min

12-14DE

Low
Temp

**FIG._2C**

**Secondary DE Summary**

FIG._3

**High Temp. Liq. Summary**

FIG._4

FIG._7

Carbohydrate Profile, Control (FRED L Liquefact)

| Hours | Glucose | Dp2 | Dp3 | >Dp3 |
|-------|---------|------|------|------|
| 16 | 94.31 | 2.28 | 0.89 | 2.51 |
| 24 | 96.71 | 1.77 | 0.67 | 0.89 |
| 40 | 96.94 | 2.09 | 0.45 | 0.52 |
| 48 | 96.93 | 2.27 | 0.39 | 0.41 |

Carbohydrate Profile, KSTM

| Hours | Glucose | Dp2 | Dp3 | >Dp3 |
|-------|---------|------|------|------|
| 16 | 95.52 | 2.18 | 0.95 | 1.35 |
| 24 | 95.55 | 2.16 | 0.94 | 1.35 |
| 41 | 95.53 | 2.18 | 0.95 | 1.35 |
| 48 | 95.69 | 2.3 | 0.87 | 1.14 |

**Effect of Residual KSTM on Saccharification**

FIG._5

Legend:
- 10 + 1 LU Added
- 10 + 0 LU Added
- 15 + 1 LU Added
- 15 + 0 LU Added

Percent Glucose

Time (In Hours)

**Effect of Liquefaction Temperature on KSTM**

FIG._6

DE

Temperature (C)

FIG._8

EP 1 435 390 B1

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- JP 10136979 B

- US 3654081 A

### Non-patent literature cited in the description

- Starch: Chemistry & Technology. **Stanley A. Watson.** Industrial Aspects. Academic Press, 1967, vol. II, 30-51

- Agricultural and Biological Chemistry. **Kanno M.** Biotechnology and Agrochem. Tokyo. 1986, vol. 50, 23-31